# EUROPEAN PATENT APPLICATION

(11) **EP 2 072 065 A1**
(43) Date of publication of application: **24.06.2009**
(21) Application number: 07025023.8
(22) Date of filing: 21.12.2007
(51) Int. Cl.: A61L 27/00, A61L 27/04, A61F 2/06, A61M 25/10

(54) **Strengthening textures in medical devices**

(71) Applicant: Abbott Laboratories Vascular Enterprises Limited, Terrace, Dublin 2 (IE)
(72) Inventor: Degen, Nicolas., 78476 Allensbach (DE); Roth, Michael., 78315 Radolfzell (DE); Ackermann, Simon., 8606 Nänikon (CH); Jayanetti, Ranil., 8200 Schaffhausen (CH); Jetter, Michael., 78247 Hilzingen (DE); Koc, Tuncay., 8222 Beringen (CH)
(74) Representative: Peters, Hajo

(57) **Abstract**

The present invention refers to medical devices. Particularly it relates to stent devices and balloon catheter devices. In the most particular aspect of the invention it relates to the strengthening of the structure of such a medical device, especially of a balloon on a balloon catheter device and its use in a variety of medical procedures to treat medical conditions in animal and human patients.

## Description

### Field of the invention

The present invention refers to medical devices. Particularly it relates to stent devices and balloon catheter devices. In the most particular aspect of the invention it relates to the strengthening of the structure of such a medical device, especially of a balloon on a balloon catheter device and it use in a variety of medical procedures to treat medical conditions in animal and human patients.

### Background of the invention

The use of stents, balloons, catheters and other medical devices etc. in minimal invasive surgery, especially in the cardiovascular field, has - over the last years - shown a high growth. As a consequence the need for modifications to the materials used fulfilling highly specialized needs in the field of different medicinal devices has clearly risen in a technical area, which traditionally is more governed by bulk products. Especially in the field of vascular balloons, especially those used in PTA (Percutaneous transluminal angioplasty) or PTCA (Percutaneous transluminal coronary angioplasty) there was a clear desire for a modified material showing a suitable compliance, especially with a flat rise in the compliance curve (pressure/diameter); and/or a high burst pressure.

With the focus of this invention on balloon material for balloon catheters one of the main parameters of a balloon is compliance, the change of the balloon diameter with rising inflation pressure; as used herein three categories are being identified:
➢ Non-compliant (NC) with a diameter increase of up to 0.55% per bar;
➢ Semi-compliant (SC) with a diameter increase of between 0.55 to 0.8 % per bar;
➢ Compliant with a diameter increase over 0.8 % per bar as the balloon is pressurized from an inflation pressure between the nominal pressure and rated burst pressure.

While a certain level of compliance is needed to allow the compression of the arterio-sclerotic plaque in a vessel, an amount of pressure expressed on the stenosis as executed by a more non-compliant balloon is also needed. As also semi-compliant and compliant balloons are more prone to failure during PTA/PTCA and also "dog-boning", an inflation of the balloon outside the stenotic area of the vessel resulting sometimes in devastating stress on the healthy part of the vessel, a more non-compliant parameter is wanted.

Another main parameter of a balloon in a balloon catheter device is burst pressure, the pressure a balloon in a balloon catheter device can withstand from within before bursting. While a certain degree of pressure expressed on the stenosis is a clear necessity for the function of a balloon catheter device the risks set to this pressure by the obviously devastating results of a possible burst of the balloon while in a lumen, e.g. of a vessel, do considerably limit the options given to a practitioner in using this device. Thus, also a high burst pressure is a strongly wanted effect in the balloon of a balloon catheter device.

A third main parameter is wall thickness. Directly related to burst pressure (and - less directly - to compliance) limited by the material used for the balloon, with the small diameters of the vessels and lumens into which such a catheter might be introduced any reduction in wall thickness is a welcome effect.

The present invention is aimed at strengthening the structure of a balloon catheter to increase the burst pressure and the controlled compliance, while also or alternatively allowing for a decrease in wall thickness. The object of the present invention is to improve the strengthened balloon structure by providing a net-structure in the inner or the outer side of the balloon made of a material with high pressure resistance. So, acting as a normal or inverted captive balloon it reduces the surface of the free unsupported area on the balloon surface exposed to pressure. This invention thus avoids a potential trade-off between increased burst pressure and controlled compliance and issues like necessary increased wall-thickness, unwanted rigidity or higher surface friction, etc. associated with prior solutions.

### Summary of the invention

The present invention is directed in one embodiment to a medical device comprising a first expandable and contractible member with an outer surface and an inner surface. In this medical device at least one second expandable member is disposed on the inner surface or outer surface of the first expandable and contractible member, which is desirably a medical balloon. The second expandable member desirably shows a net-like structure and/or is bound to the first expandable and contractible member by melting or gluing.

In another embodiment the invention is further directed to a medical device being a delivery apparatus for delivering at least one second medical device comprising a first expandable and contractible member with an outer surface and an inner surface. The second medical device is an expandable medical device disposed about the first expandable and contractible member and desirably is a stent. In this medical device at least one second expandable member is disposed on the inner or outer surface of the first expandable and contractible member, which is desirably a medical balloon. The second expandable member desirably shows a net-like structure and/or is bound to the first expandable and contractible member by melting or gluing.

In yet another embodiment the invention is directed to a method for improving the burst pressure of a first expandable and contractible member of a medical device comprising: providing a first expandable and contractible member with an outer surface and an inner surface; and disposing a second expandable member on the inner or outer surface of the first expandable and contractible member. The second expandable member desirably shows a net-like structure and/or is bound to the first expandable and contractible member- which desirably is a medical balloon - by melting or gluing.

In a further embodiment the invention is directed to a method of producing a medical device according to the invention. In this method a second expandable member is disposed on the inner surface of a first expandable and contractible member by expanding a third expandable and contractible member disposed inside the second expandable member, thus pressing the second expandable member onto the inner surface of the first expandable and contractible member. Desirably the second expandable member is bound to the first expandable and contractible member by melting, by e.g. heating the first expandable and contractible member and/or the second expandable member until at least part of the surface of one or both of them are melted before pressing them together or by gluing.

In a further embodiment the invention is directed to a method of treatment of a disease, like a cardiovascular disease, especially a stenosis, using in a patient, being a mammal, especially a human, in need thereof a medical device according to the invention, desirably in minimal invasive surgery like PTA or PTCA.

In yet another embodiment the invention is directed to the use of a medical device according to the invention for the treatment of a disease, like a cardiovascular disease, especially a stenosis, especially through minimal invasive surgery like PTA or PTCA.

### Brief Description of the Drawings:

- **Figure A**: depicts a medical device (D) according to the invention with the Inner Surface (10) and the Outer Surface (11) of the first expandable and contractible member (B) and the Second expandable member (7).

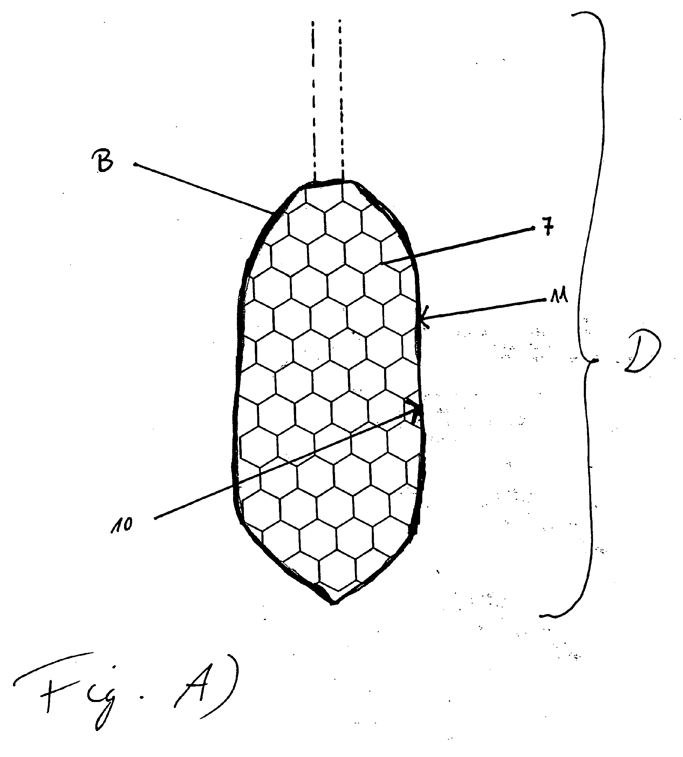

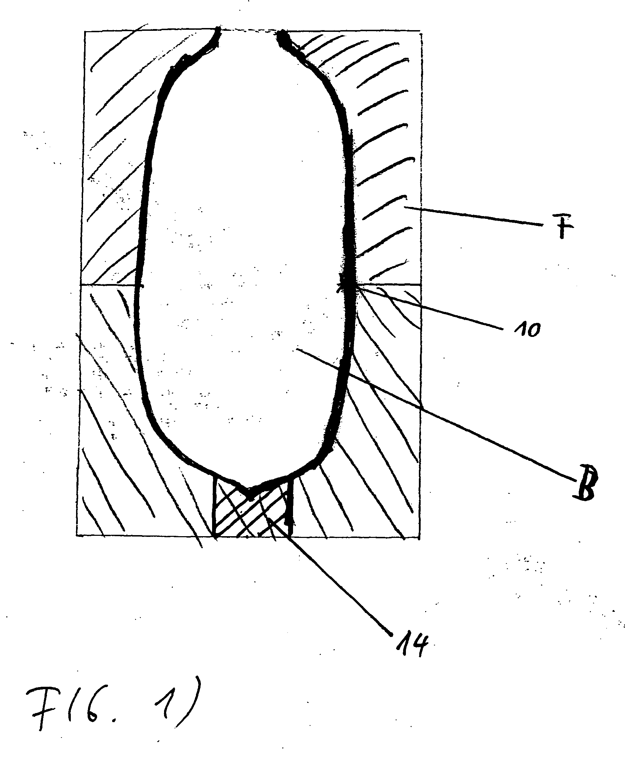

- **Figures 1**: **to 6** illustrate a preferred embodiment of a medical device according to the invention exemplified along its production process, focussing on the production of the First expandable member (B) with the Second expandable member (7) disposed on its inner surface (10). In this example described in more details below:
• The first expandable and contractible member (B) is exemplified as a Medical Balloon.
• The second expandable member (7) is exemplified as a Net-like Structure.
• The third expandable and contractible member (5) is exemplified as a Secondary Balloon.
- **Figure 1**: depicts a medical balloon (B) (the First expandable and contractible member) blown in a blow molding form (F), with the inner surface (10) of the balloon also being shown. Fig. 1) refers to the 1^{st} Step of the example below. Also shown is the optional second aperture (14) usual in most of the blow molding forms (F).

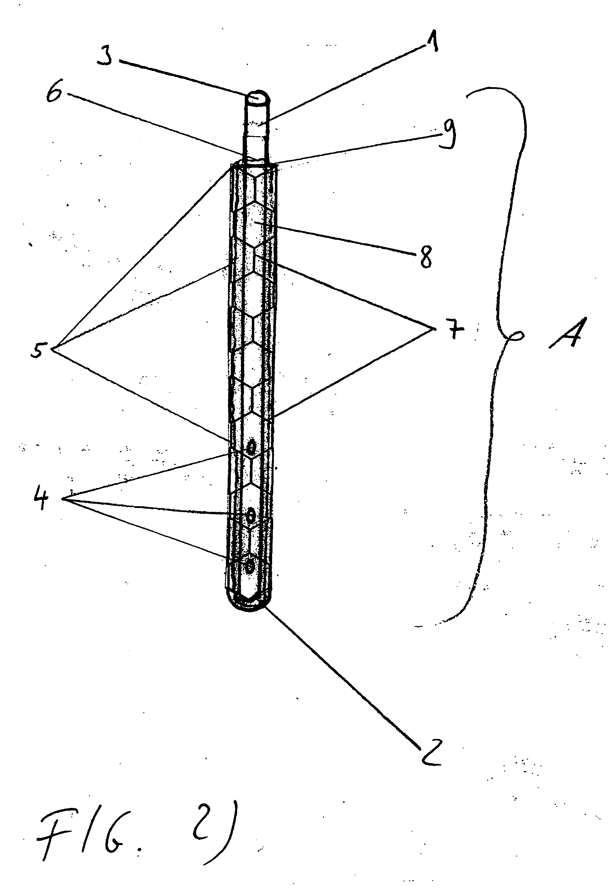

- **Figure 2**: depicts the Construct (A). In the core it shows a needle (1) in form of a tube with a hollow core (3) with a blended tip (2) and holes (4) connected to the inner hollow core (3) of the tube. It also shows as next layer a secondary balloon (5) (the third expandable and contractible member) imposed on the needle (1) with its upper rim (6) air-tightly fixed about the upper part of needle (1). It further shows as top layer a net-like structure (7) (the second expandable member) of hexagonal shaped spaces (8) imposed on the secondary balloon (5) and attached with its upper end (9) to the upper part of the needle (1). Fig. 2) refers to the 2^{nd} Step of the example below.

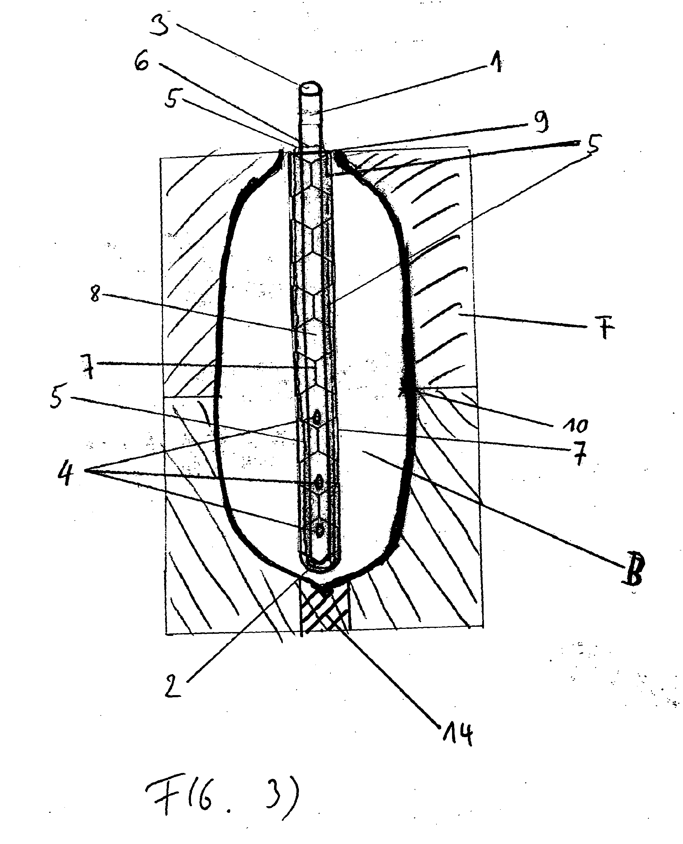

- **Figure 3**: depicts the Construct (A) being introduced into the blow molding form (F) and the medical balloon (B). Fig. 3) refers to the 3^{rd} Step of the example below. Also shown is the optional second aperture (14) usual in most of the blow molding forms (F).

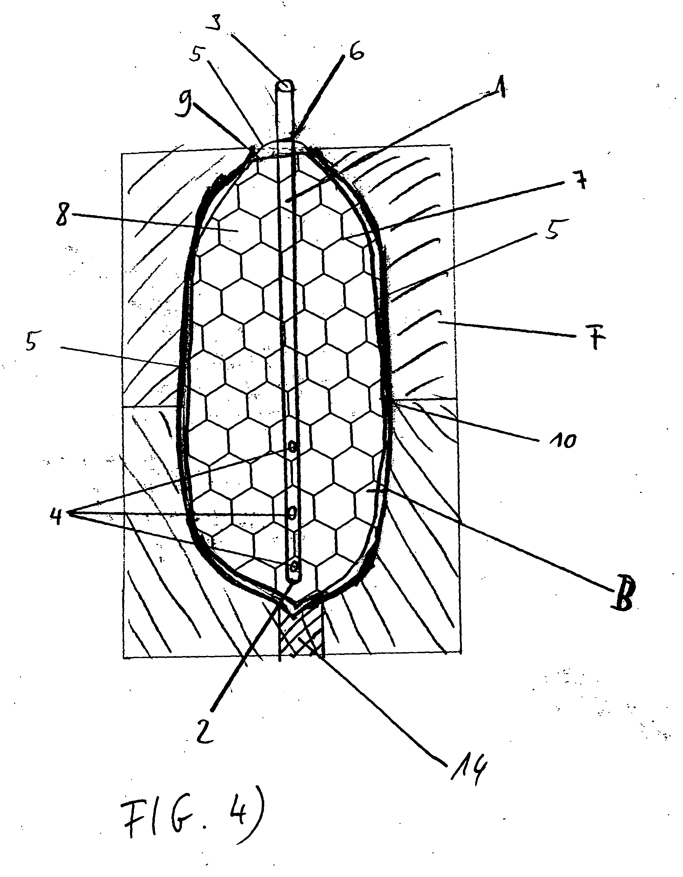

- **Figure 4**: depicts the secondary balloon (5) being inflated to press the net-like structure (7) onto the inner surface (10) of the medical balloon (B). The blow molding form (F) heated to melt the inner surface (10) of the medical balloon (B) and the hexagonal (8) shaped net-like structure (7) provides the counter pressure. The secondary balloon (5) is inflated by hot air through the inner hollow core (3), the blended tip (2) and the holes (4) of the needle (1) of Construct (A). Fig. 4) refers to the 4^{th} Step (a) and (b) of the example below. Also shown is the optional second aperture (14) usual in most of the blow molding forms (F).

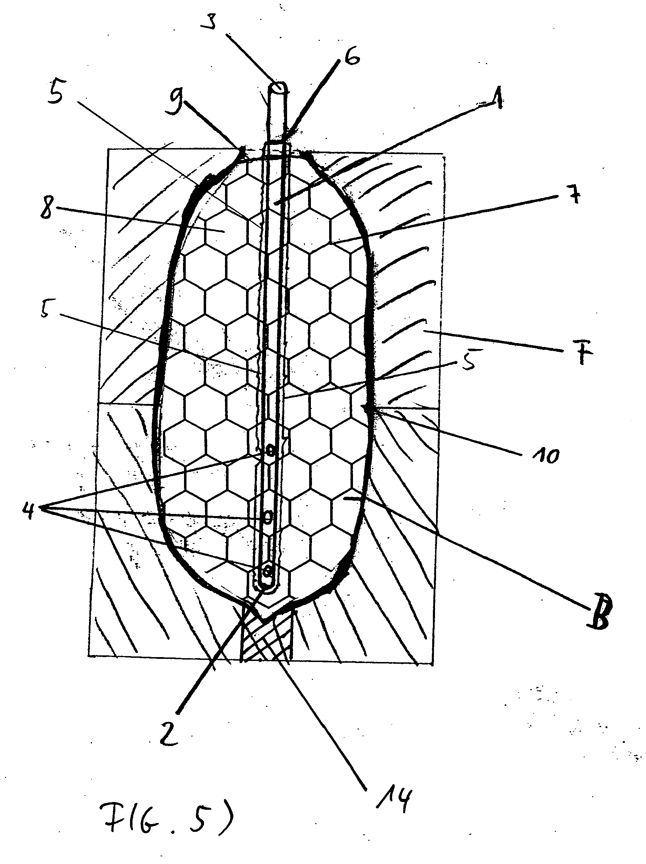

- **Figure 5**: depicts the deflated secondary balloon (5) with the net-like structure (7) remaining expanded and bound onto the inner surface (10) of the medical balloon (B). Fig. 5) refers to the 4^{th} Step (c) of the example below before removal of needle (1) and secondary balloon (5) from within the blow molding form (F). Also shown is the optional second aperture (14) usual in most of the blow molding forms (F).

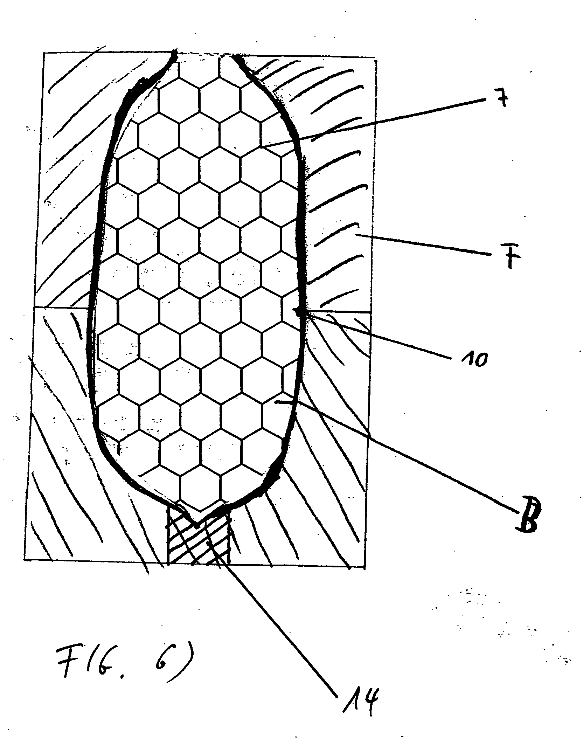

- **Figure 6**: depicts the medical balloon (B) reinforced with the net-like structure (7) remaining bound onto the inner surface (10) of the medical balloon (B). Fig. 6) refers to the 5^{th} Step of the example below before removal of the medical balloon (B) from the molding form (F). Also shown is the optional second aperture (14) usual in most of the blow molding forms (F).

### Detailed Description of the Invention

While the invention may be embodied in many different forms, there are described in detail herein specific preferred embodiments of the invention. This description is an exemplification of the principles of the invention and is not intended to limit the invention to the particular embodiments illustrated.

In one embodiment the instant invention is directed to a medical device (D) such as shown generally in Fig. A). This medical device comprises a first expandable and contractible member (B) with an outer surface (11) and an inner surface (10); wherein a second expandable member (7) is disposed on the inner surface (10) or on the outer surface (11) of the first expandable and contractible member (B). As one alternative desirably the second expandable member (7) is disposed on the inner surface (10) or on the outer surface (11) of the first expandable and contractible member (B). As another alternative desirably the second expandable member (7) is disposed on the outer surface (11) of the first expandable and contractible member (B).

In this embodiment the expandable and contractible member (B) desirably is a medical balloon. The medical balloon (B) is capable of being expanded and contracted. The medical balloon may also be made of any balloon material known in the art. Examples of Balloon material may be found is U.S. Pat. Nos. 6,406,457 B1, 5,830,182 A, 6,171,278 B1, 5,556,383 and 6,924,753 and elsewhere in the patent literature. Desirably the first expandable and contractible member (B), especially the medical balloon, is consisting of a polymer, desirably selected from Nylon, PEBA or mixtures thereof.

In this embodiment desirably the second expandable member (7) is disposed only in partial areas of the inner surface (10) or outer surface (11) of the first expandable and contractible member (B), preferably is disposed only in the median partial areas or the cylindrical portion of the inner surface (10) or outer surface (11) of the first expandable and contractible member (B). Therefore, the advantage of increased strength transferred by the second expandable member (7) to the first expandable and contractible member (B) is directed to the areas of the first expandable and contractible member (B) which mostly need strengthening e.g. the median (central) partial area or the cylindrical portion of a balloon upon expansion.

In this embodiment of the medical device according to the invention desirably the second expandable member (7) has at least one perforated layer or surface, desirably being homogeneous or is formed from various strands. Especially the second expandable member (7) has a net-like structure, preferably a hexagonal net-like structure. The net-like structure (7) is expandable and by adhering to the inner surface (10) or the outer surface (11) of the first expandable and contractible member (B) it also further reduces the surface of the free unsupported areas of the first expandable and contractible member (B), especially if this is an inflated Medical Balloon (B). Desirably the net-like structure is a comb-like net, especially a cascaded hexagon or with hexagonal shapes (8) thus helping to support/reinforce the first expandable and contractible member/Medical Balloon (B).
This support has the big advantage that, while maintaining the same burst pressure, this would also allow to thin-down the wall thickness of the first expandable and contractible member/Medical Balloon (B) or respectively would allow to maintain the wall-thickness while increasing burst pressure, which is highly desirably allowing a smaller size of the folded first expandable and contractible member/Medical Balloon (B) giving an advantage when using this medical device in a very small lumen.

Further to this embodiment of the medical device according to the invention desirably the second expandable member (7) is consisting of a polymer or nanotubes. Nanotubes are nanometer scale tubular structures that can be grown (e.g. on the surface of a material (later) forming the first expandable and contractible member (B)) and in principle may be carbon nanotubes or inorganic nanotubes. If the second expandable member (7) is consisting of a polymer desirably the polymer is selected from Nylon or other high-strength polymers, desirably is a polymer selected from polyethylene terephtalate (PET), polyurethane or a polyamide, preferably is Nylon. Nylon does show the advantage that it has a high tensile strength, which is advantageous in that it confers a controlled compliance and increased burst pressure to be achieved by the current invention. In some embodiments it may be desirable to include particles in the polymer that are susceptible to microwave like metal parts. When bonding the second expandable member (7) to the first expandable and contractible member (B) applying microwaves results in the second expandable member (7) melting to the surface of the first expandable and contractible member (B).

Further to this embodiment of the medical device according to the invention desirably the second expandable member (7) is bonded to the first expandable and contractible member (B) by melting or by gluing with an adhesive.
In the variant using melting this thermal welding could be achieved by heating the second expandable member (7) and/or the first expandable and contractible member (B) until the surface of one or both of them start to melt and then pressing one of them onto the other.
In the variant using gluing with an adhesive, the adhesive preferably is a flexible adhesive, with the adhesive being applied onto the second expandable member (7) and/or onto the first expandable and contractible member (B).
In both variants the bonding is desirably achieved by expansion of a third expandable and contractible member (5), desirably a secondary balloon, inside the second expandable member (7), especially of net-like structure, thus pressing the second expandable member (7) onto the inner surface (10) of the first expandable and contractible member (B). Desirably this third expandable and contractible member (5) is made of a polymer, a material with a higher melting point than the material the second expandable member (7) and/or the first expandable and contractible member (B), and thus showing a high thermal resistance and a low wettability or being not-wettable. Desirably the third expandable and contractible member (5) is made of polytetrafluoroethylene.

In another embodiment the second expandable member (7) is not bonded to the first expandable and contractible member (B).

In another variant for of the embodiment of the medical device according to the invention with the second expandable member (7) being bonded to the first expandable and contractible member (B) by melting the second expandable member (7) comprises particles that are susceptible to microwaves, like metal parts, e.g. in the polymer. Accordingly after the second expandable member (7) is brought in contact with the first expandable and contractible member (B) microwaves are applied resulting in that the second expandable member (7) is melting to the surface of the first expandable and contractible member (B).

Further to the embodiment of the medical device according to the invention desirably a fourth expandable and contractible member (12) is disposed inside the inner surface (10) of the first expandable and contractible member (B) and inside the second expandable member (7). In this embodiment thus the second expandable member (7) is inside a sandwich-like structure but still transferring its strengthening to the first expandable and contractible member (B) and the fourth expandable and contractible member (12).

In one embodiment the medical device (D) is a delivery apparatus for delivering at least one second medical device. Desirably the medical device is a delivery apparatus with a stent, a stent graft, a graft or a graft connector as second medical device. Very desirably the medical device is a delivery apparatus with a stent as second medical device. In one embodiment this delivery apparatus comprises a catheter with a medical balloon (B) as first expandable and contractible member. Thereby the second medical device, the stent, is disposed about the medical balloon (B) (the first expandable and contractible member). Additional details concerning the construction of suitable stent delivery apparatuses for use in the invention may be found in U.S. Pat. Nos. 6,036,697, 5,893,868 and 5,957,930 and elsewhere in the patent literature. Any suitable stent may be used whether formed of metal or of polymeric material or of another material. Examples of suitable stents may be found in US 6,602,285 and US 6,533,809. The Medical balloon is capable of being expanded and contracted. The Medical balloon may be made of any balloon material known in the art. Examples of Balloon material may be found is U.S. Pat. Nos. 6,406,457 B1, 5,830,182 A, 6,171,278 B1, 5,556,383 and 6,924,753 and elsewhere in the patent literature. Desirably the first expandable and contractible member (B), especially the medical balloon, is consisting of a polymer, desirably selected from Nylon, PEBA or mixtures thereof.

"Balloon", "Medical Balloon" or "balloon material" in the context of this invention especially means a balloon like those used in balloon angioplasty and the material used for these balloons, especially balloon catheters. In this, e.g. a balloon catheter is inserted into an artery or other lumen and advanced to e.g. a narrowing in a coronary artery. The balloon is then inflated by gas or fluids to enlarge the lumen and/or - often - to place a medical device.

"Stent" means an elongate implant with a hollow interior and at least two orifices and usually a circular or elliptical, but also any other, cross section, preferably with a perforated, lattice-like structure that is implanted into vessels, in particular blood vessels, to restore and maintain the vessels patent and functional.

"Graft" means an elongate implant with a hollow interior and with at least two orifices and usually circular or elliptical, but also any other, a cross section and with at least one closed polymer surface which is homogeneous or, optionally, woven from various strands. The surface preferably is impermeable to corpuscular constituents of blood and/or for water, so that the implant serves as a vascular prosthesis and is usually employed for damaged vessels or in place of vessels.

"Stent graft" means a connection between a stent and a graft. A stent graft preferably comprises a vascular prosthesis reinforced with a stent (both as defined above), wherein a polymer layer is homogeneous or, optionally, woven, knitted plaited etc. from various strands and is either impermeable for corpuscular constituents of blood and/or for water or can also be permeable. More preferably, the stent has on at least 20% of its surface a perforated (lattice-like), preferably metallic, outer layer and at least one closed polymer layer that is located inside or outside the stent outer layer. The closed polymer layer may be homogeneous or, optionally, woven from various strands, and is impermeable for corpuscular constituents of blood and/or for water. Optionally, where the closed polymer layer is disposed inside the metallic outer layer, a further perforated (lattice-like), preferably metallic, inner layer may be located inside the polymer layer.

"Graft connector" means an implant that connects at least two hollow organs, vessels or grafts, consists of the materials defined for grafts or stent grafts and/or has the structure defined for the latter. Preferably, a graft connector has at least two, three or four, orifices, arranged, for example, as an asymmetric "T" shape.

"Catheter" means a tubular instrument intended for introduction into hollow organs. More preferably, a catheter may be designed for use in guiding other catheters, or for angiography, ultrasound imaging, or - especially - balloon catheters for dilatation or stent delivery. This includes also a "Catheter pump" meaning a catheter provided on its tip with a propeller able to assist the pumping of the myocardium.

In this embodiment of the medical device as a delivery apparatus for delivering at least one second medical device, especially a stent, according to the invention desirably the second expandable member (7) has at least one perforated layer or surface, desirably being homogeneous or is formed from various strands. Especially the second expandable member (7) has a net-like structure, preferably a hexagonal net-like structure. The net-like structure (7) is expandable and by adhering to the inner surface (10) or outer surface (11) of the first expandable and contractible member (B) it also further reduces the surface the free unsupported areas of the first expandable and contractible member (B), especially if this is an inflated Medical Balloon (B) of a stent delivery apparatus. Desirably the net-like structure is a comb-like net, especially a cascaded hexagon or with hexagonal shapes (8) thus helping to support/reinforce the first expandable and contractible member/Medical Balloon (B).

This support has the big advantage that, while maintaining the same burst pressure, this would also allow to thin-down the wall thickness of the first expandable and contractible member/Medical Balloon (B) or respectively would allow to maintain the wall-thickness while increasing burst pressure, especially of a Medical Balloon (B) of a stent delivery apparatus, which is highly desirably allowing a smaller size of the folded first expandable and contractible member/Medical Balloon (B) giving an advantage when using this medical device in a very small lumen.

Further to this embodiment of the medical device as a delivery apparatus for delivering at least one second medical device, especially a stent, according to the invention desirably the second expandable member (7) is consisting of a polymer or nanotubes. Nanotubes are nanometer scale tubular structures that can be grown (e.g. on the surface of a material (later) forming the first expandable and contractible member (B)) and in principle may be carbon nanotubes or inorganic nanotubes. If the second expandable member (7) is consisting of a polymer desirably the polymer is selected from Nylon or other high-strength polymers, desirably is a polymer selected from polyethylene terephtalate (PET), polyurethane or a polyamide, preferably is Nylon, desirably selected from Nylon or other high-strength polymers. Nylon does show the advantage that it has a high tensile strength, which is advantageous in that it confers a controlled compliance and increased burst pressure to be achieved by the current invention, especially to a Medical Balloon (B) of a stent delivery apparatus.

Further to this embodiment of the medical device as a delivery apparatus for delivering at least one second medical device, especially a stent, according to the invention desirably the second expandable member (7) is disposed only in partial areas of the inner surface (10) or outer surface (11) of the first expandable and contractible member (B), preferably is disposed only in the median partial areas or the cylindrical portion of the inner surface (10) or outer surface (11) of the first expandable and contractible member (B). Therefore, the advantage of increased strength transferred by the second expandable member (7) to the first expandable and contractible member (B), the medical balloon, is directed to the areas of the first expandable and contractible member (B) which mostly need strengthening e.g. the median (central) partial area or the cylindrical portion of a balloon - the area carrying the stent - upon expansion.

Further to this embodiment of the medical device as a delivery apparatus for delivering at least one second medical device, especially a stent, according to the invention desirably a fourth expandable and contractible member (12) is disposed inside the inner surface (10) of the first expandable and contractible member (B) and inside the second expandable member (7). In this embodiment thus the second expandable member (7) is inside a sandwich-like structure but still transferring it's strengthening to the first expandable and contractible member (B) and the fourth expandable and contractible member (12).

Further to this embodiment of the medical device as a delivery apparatus for delivering at least one second medical device, especially a stent, according to the invention, desirably the second expandable member (7) is bound to the first expandable and contractible member (B) by melting or by gluing with an adhesive.
In the variant using melting this thermal welding could be achieved by heating the second expandable member (7) and/or the first expandable and contractible member (B) until the surface of one or both of them start to melt and then pressing one of them onto the other.
In the variant using gluing with an adhesive, the adhesive preferably is a flexible adhesive, with the adhesive being applied onto the second expandable member (7) and/or onto the first expandable and contractible member (B).
In both variants the bonding is desirably achieved by expansion of a third expandable and contractible member (5), desirably a secondary balloon, inside the second expandable member (7), especially of net-like structure, thus pressing the second expandable member (7) onto the inner surface (10) of the first expandable and contractible member (B). Desirably this third expandable and contractible member (5) is made of a polymer, a material with a higher melting point than the material the second expandable member (7) and/or the first expandable and contractible member (B), and thus showing a high thermal resistance and a low wettability or being not-wettable. Desirably the third expandable and contractible member (5) is made of polytetrafluoroethylene.
In another embodiment the second expandable member (7) is not bonded to the first expandable and contractible member (B).

Another aspect and embodiment of the current invention is directed to a method for improving the burst pressure of a first expandable and contractible member (B) of a medical device (D) comprising: providing a first expandable and contractible member (B) with an outer surface (11) and an inner surface (10), and disposing a second expandable member (7) on the inner surface (10) or the outer surface (11) of the first expandable and contractible member (B). As one alternative desirably the second expandable member (7) is disposed on the inner surface (10) or on the outer surface (11) of the first expandable and contractible member (B). As another alternative desirably the second expandable member (7) is disposed on the outer surface (11) of the first expandable and contractible member (B). In this embodiment desirably the first expandable and contractible member (B) is a medical balloon. The Medical balloon (B) is capable of being expanded and contracted. The Medical balloon may also be made of any balloon material known in the art. Examples of Balloon material may be found is U.S. Pat. Nos. 6,406,457 B1, 5,830,182 A, 6,171,278 B1, 5,556,383 and 6,924,753 and elsewhere in the patent literature. Desirably the first expandable and contractible member (B), especially the medical balloon, is consisting of a polymer, desirably selected from Nylon, PEBA or mixtures thereof. Also in this embodiment desirably the second expandable member (7) is disposed only in partial areas of the inner surface (10) or outer surface (11) of the first expandable and contractible member (B), preferably is disposed only in the median partial areas or the cylindrical portion of the inner surface (10) or outer surface (11) of the first expandable and contractible member (B). Therefore, the advantage of increased strength transferred by the second expandable member (7) to the first expandable and contractible member (B) is directed to the areas of the first expandable and contractible member (B) which mostly need strengthening e.g. the median (central) partial area or the cylindrical portion of a balloon upon expansion.

In this embodiment of a method for improving the burst pressure of a first expandable and contractible member (B) of a medical device (D) according to the invention desirably the second expandable member (7) has at least one perforated layer or surface, desirably being homogeneous or is formed from various strands. Especially the second expandable member (7) has a net-like structure, especially has a hexagonal net-like structure. The net-like structure (7) is expandable and by adhering to the inner surface (10) or the outer surface (11) of the first expandable and contractible member (B) it also further reduces the surface of the free unsupported areas of the first expandable and contractible member (B), especially if this is an inflated Medical Balloon (B). Desirably the net-like structure is a comb-like net, especially a cascaded hexagon or with hexagonal shapes (8) thus helping to support/reinforce the first expandable and contractible member/Medical Balloon (B).

This support has the big advantage that, while maintaining the same burst pressure, this would also allow to thin-down the wall thickness of the first expandable and contractible member/Medical Balloon (B) or respectively would allow to maintain the wall-thickness while increasing burst pressure, which is highly desirably allowing a smaller size of the folded first expandable and contractible member/Medical Balloon (B) giving an advantage when using this medical device in a very small lumen.

Further to this embodiment of a method for improving the burst pressure of a first expandable and contractible member (B) of a medical device (D) according to the invention desirably the second expandable member (7) is consisting of a polymer or nanotubes. Nanotubes are nanometer scale tubular structures that can be grown (e.g. on the surface of a material (later) forming the first expandable and contractible member (B)) and in principle may be carbon nanotubes or inorganic nanotubes. If the second expandable member (7) is consisting of a polymer desirably the polymer is selected from Nylon or other high-strength polymers, desirably is a polymer selected from polyethylene terephtalate (PET), polyurethane or a polyamide, preferably is Nylon, desirably selected from Nylon or other high-strength polymers. Nylon does show the advantage that it has a high tensile strength, which is advantageous in that it confers a controlled compliance and increased burst pressure to be achieved by the current invention.

Further to this embodiment of a method for improving the burst pressure of a first expandable and contractible member (B) of a medical device (D) according to the invention desirably the second expandable member (7) is bonded to the first expandable and contractible member (B) by melting. This thermal welding could be achieved by heating (beyond melting temperature) the second expandable member (7) until its surface starts to melt and then pressing it onto the first expandable and contractible member (B); or by heating (beyond melting temperature) the first expandable and contractible member (B) until the surface of it starts to melt and then pressing the second expandable member (7) onto it; or by heating(beyond melting temperature) the second expandable member (7) and the first expandable and contractible member (B) until the surface of one or both of them start to melt and then pressing one of them onto the other. This is desirably achieved by expansion of a third expandable and contractible member (5), desirably a secondary balloon, inside the second expandable member (7), especially of net-like structure, thus pressing the second expandable member (7) onto the inner surface (10) of the first expandable and contractible member (B). Desirably this third expandable and contractible member (5) is made of a polymer, a material with a higher melting point than the material the second expandable member (7) and/or the first expandable and contractible member (B), and thus showing a high thermal resistance and a low wettability or being not-wettable. Desirably the third expandable and contractible member (5) is made of polytetrafluoroethylene

Further to this embodiment of a method for improving the burst pressure of a first expandable and contractible member (B) of a medical device (D) according to the invention desirably the second expandable member (7) is bonded to the first expandable and contractible member (B) by gluing with an adhesive, preferably a flexible adhesive. This is achieved by applying the adhesive
either
onto the second expandable member (7) which is then pressed onto the inner surface (10) of the first expandable and contractible member (B);
or
onto the first expandable and contractible member (B) before the second expandable member (7) is pressed onto the inner surface (10) of the first expandable and contractible member (B);
or
onto the first expandable and contractible member (B) and the second expandable member (7) before the second expandable member (7) is pressed onto the inner surface (10) of the first expandable and contractible member (B).

The pressure onto each other is desirably achieved by expansion of a third expandable and contractible member (5), desirably a secondary balloon, inside the second expandable member (7), especially of net-like structure, thus pressing the second expandable member (7) onto the inner surface (10) of the first expandable and contractible member (B). Desirably this third expandable and contractible member (5) is made of a polymer, a material with a higher melting point than the material the second expandable member (7) and/or the first expandable and contractible member (B), and thus showing a high thermal resistance and a low wettability or being not-wettable. Desirably the third expandable and contractible member (5) is made of polytetrafluoroethylene

In one further embodiment of a method for improving the burst pressure of a first expandable and contractible member (B) of a medical device (D) the medical device (D) is a delivery apparatus for delivering at least one second medical device. Desirably the medical device is a delivery apparatus with a stent, a stent graft, a graft or a graft connector as second medical device. Very desirably the medical device is a delivery apparatus with a stent as second medical device. In one embodiment of a method for improving the burst pressure of a first expandable and contractible member (B) of a medical device (D) this delivery apparatus comprises a catheter with a medical balloon (B) as first expandable and contractible member. Thereby the second medical device, the stent, is disposed about the medical balloon (B) (the first expandable and contractible member). Additional details concerning the construction of suitable stent delivery apparatuses for use in the invention may be found in U.S. Pat. Nos. 6,036,697, 5,893,868 and 5,957,930 and elsewhere in the patent literature. Any suitable stent may be used whether formed of metal or of polymeric material or of another material. Examples of suitable stents may be found in US 6,602,285 and US 6,533,809. The Medical balloon is capable of being expanded and contracted. The Medical balloon may be made of any balloon material known in the art. Examples of Balloon material may be found is U.S. Pat. Nos. 6,406,457 B1, 5,830,182 A, 6,171,278 B1, 5,556,383 and 6,924,753 and elsewhere in the patent literature. Desirably the first expandable and contractible member (B), especially the medical balloon, is consisting of a polymer, desirably selected from Nylon, PEBA or mixtures thereof.

Another aspect and embodiment of the current invention is directed to a method of producing a medical device according to the invention, wherein a second expandable member (7) is disposed on the inner surface (10) of a first expandable and contractible member (B) by expanding a third expandable and contractible member (5) disposed inside the second expandable member (7), thus pressing the second expandable member (7) onto the inner surface (10) of the first expandable and contractible member (B). Desirably in variant a) thermally welding is employed in that the second expandable member (7) and/or the first expandable and contractible member (B) are heated up to their melting temperature and by expanding the third expandable and contractible member (5) disposed inside the second expandable member (7) after at least part of the surface of one or both of the second expandable member (7) or the first expandable and contractible member (B) the second expandable member (7) is pressed onto the inner surface (10) of the first expandable and contractible member (B). Also desirably in variant b) an adhesive, preferably a flexible adhesive is applied onto the second expandable member (7) and/or the first expandable and contractible member (B) and the third expandable and contractible member (5) disposed inside the second expandable member (7) is expanded, so that the second expandable member (7) is pressed onto the inner surface (10) of the first expandable and contractible member (B).
Desirably in both variants this third expandable and contractible member (5) is made of a polymer, a material with a higher melting point than the material the second expandable member (7) and/or the first expandable and contractible member (B), and thus showing a high thermal resistance and a low wettability or being not-wettable. Desirably the third expandable and contractible member (5) is made of polytetrafluoroethylene

Another aspect and embodiment of the current invention is directed to a method of producing a medical device according to the invention, wherein a second expandable member (7) is disposed on the outer surface (11) of a first expandable and contractible member (B) by pulling the second expandable member (7) over at least a partial area of the outer surface (11) of the first expandable and contractible member (B) and optionally bonding the second expandable member (7) to the surface (11) of the first expandable and contractible member (B) by melting or gluing with an adhesive.

Another aspect and embodiment of the current invention is directed to a method of treatment of a disease, like a cardiovascular disease, especially a stenosis, using in a patient, being a mammal, especially a human, in need thereof a medical device (D) according to the invention, desirably in minimal invasive surgery like PTA or PTCA. In this, the first expandable and contractible member (B) (the Medical Balloon) being reinforced by the second expandable member (7) (the net-like structure) can be advantageously used, having an improved/controlled compliance and higher burst pressure or lower wall-thickness.

A further aspect and embodiment of the current invention is directed to the use of a medical device (D) according to the invention for the treatment of a disease, like a cardiovascular disease, especially a stenosis, especially through minimal invasive surgery like PTA or PTCA. In this, the first expandable and contractible member (B) (the Medical Balloon) being reinforced by the second expandable member (7) (the net-like structure) can be advantageously used, having an improved/controlled compliance and higher burst pressure or lower wall-thickness.

### Example:

**Production Process of the Medical Device according to the invention** focussing on the production of the first expandable and contractible member (B) being exemplified as a Medical Balloon reinforced by the second expandable member (7) exemplified as a Net-like Structure.

### 1^{st} Step: Blowing of the Medical Balloon (B)

According to standard procedures known in the art a medical balloon (B) formed from Nylon-12 is blown in a blow molding form (F). Following that the balloon is ready-for-use, but is not yet removed.

### 2^{nd} Step: Construction of the Construct (A):

A) A needle (1) with a blended tip (2) forms the middle axis of the Construct (A). The needle consists of metal and is in form of a tube with a hollow core (3) in connection with or connectable to a source of gas and holes (4) connected to the inner hollow core (3) of the tube.
B) A secondary balloon (5) consisting of Teflon is imposed on the needle (1) covering the blended tip (2), being with its upper rim (6) air-tightly fixed about the upper part of needle (1).
C) A net-like structure (7) of hexagonal shaped spaces (8) consisting of Nylon 12 is imposed on the secondary balloon (5) and attached - but not fixed - with its upper end (9) to the upper part of the needle (1).

### 3^{rd} Step: Introduction of Construct (A) into Medical Balloon (B)

The construct (A) is introduced into the pre-shaped medical balloon (B) resting in the blow molding form (F).

### 4^{th} Step: Disposing of the net-structure (7) on the inner Surface (10) of Medical Balloon (B)

a) The blow molding form (F) is heated up to a temperature at which the net-like structure (7) of construct (A) and the Medical Balloon (B), especially its inner surface (10) get melted on the surface.
b) Hot Air is introduced through the hollow core (3), the tip (2) and the holes (4) of the needle (1) and the secondary balloon (5) is inflated. By this the secondary balloon (5) presses the net-structure (/) onto the inner surface (10) of the medical balloon (B). As the net-like structure (7) and the inner surface (10) both were heated until their surface bet melted, pressing them together results in the net-like structure (7) being rigidly bound to the inner surface (10) of the Medical Balloon (B).
c) The blow molding form (F) is cooled-down. Following that the secondary balloon (5) is deflated by pumping air out of the secondary balloon (5) through the hollow core (3), the tip (2) and the holes (4) of the needle (1). Following that the Construct (A) without the net-like structure (/) is removed from within the Medical Balloon (B) and the blow molding form (F).

### 5^{th} Step: Removal of the reinforced Medical Balloon (B)

The Medical Balloon (B) - now reinforced by the net-like structure (7) bound to the inner surface (10) - is removed from the blow molding form.

The advantages of the reinforced medical balloons are shown when the balloon according to the invention is tested using burst-pressure and compliance tests known in the art.

## Claims

1. A medical device (D) comprising:
a first expandable and contractible member (B) with an outer surface (11) and an inner surface (10);
wherein a second expandable member (7) is disposed on the inner surface (10) or the outer surface (11) of the first expandable and contractible member (B).

2. A medical device according to claim 1, wherein the second expandable member (7) is disposed on the inner surface (10) of the first expandable and contractible member (B).

3. A medical device according to claim 1, wherein the second expandable member (7) is disposed on the outer surface (10) of the first expandable and contractible member (B).

4. The medical device according to any of claims 1 to 3, wherein the second expandable member (7) is disposed only in partial areas of the inner surface (10) or outer surface (11) of the first expandable and contractible member (B), preferably is disposed only in the median partial areas or the cylindrical portion of the inner surface (10) or outer surface (11) of the first expandable and contractible member (B).

5. The medical device according to any of claims 1 to 4, wherein the expandable and contractible member (B) is a medical balloon.

6. The medical device according to any of claims 1 to 5, wherein the first expandable and contractible member (B) is consisting of a polymer, preferably is consisting of a polymer selected from Nylon, PEBA or mixtures thereof.

7. The medical device according to any of claims 1 to 6, wherein the second expandable member (7) is consisting of a polymer or nanotubes.

8. The medical device according to claim 7, wherein the polymer is polyethylene terephtalate (PET), polyurethane or a polyamide, preferably is Nylon.

9. The medical device according to any of claims 1 to 7, wherein the second expandable member (7) has at least one perforated layer or surface; preferably has at least one perforated layer or surface being homogeneous or being formed from various strands.

10. The medical device according to any of claims 1 to 9, wherein the second expandable member (7) has a net-like structure, preferably has a hexagonal net-like structure.

11. The medical device according to any of claims 1 to 10, wherein the second expandable member (7) is bound to the first expandable and contractible member (B) by melting or by gluing with an adhesive, preferably is bound to the first expandable and contractible member (B) by melting or by gluing with a flexible adhesive.

12. The medical device according to any of claims 1 or 2, wherein a fourth expandable and contractible member (12) is disposed inside the inner surface (10) of the first expandable and contractible member (B) and inside the second expandable member (7).

13. The medical device according to any of claims 1 to 12, wherein the medical device (D) is a delivery apparatus for delivering at least one second medical device.

14. The medical device according to claim 12 comprising as second medical device an expandable medical device disposed about the first expandable and contractible member (B).

15. The medical device according to claim 14, wherein the expandable medical device is a stent, a stent graft, a graft or a graft connector, preferably is a stent .

16. A method for improving the burst pressure of a first expandable and contractible member (B) of a medical device (D) comprising:
providing a first expandable and contractible member (B) with an outer surface (11) and an inner surface (12);
disposing a second expandable member (7) either on the inner surface (10) or on the outer surface (11) of the first expandable and contractible member (B).

17. The method according to claim 16, wherein the second expandable member (7) is disposed on the inner surface (10) of the first expandable and contractible member (B).

18. The method according to claim 16, wherein the second expandable member (7) is disposed on the outer surface (11) of the first expandable and contractible member (B).

19. The method according to any of claims 16 to 18, wherein the first expandable and contractible member (B) is a medical balloon.

20. The method according to any of claims 16 to 19, wherein the first expandable and contractible member (B) is consisting of a polymer, preferably is consisting of a polymer selected from Nylon, PEBA or mixtures thereof.

21. The method according to any of claims 16 to 20, wherein the second expandable member (7) is consisting of a polymer or nanotubes.

22. The method according to any of claims 21, wherein the polymer is polyethylene terephtalate (PET), polyurethane or a polyamide, preferably is Nylon.

23. The method according to any of claims 16 to 22, wherein the second expandable member (7) has at least one perforated layer or surface; preferably has at least one perforated layer or surface being homogeneous or being formed from various strands.

24. The method according to any of claims 16 to 23, wherein the second expandable member (7) has a net-like structure, preferably has a hexagonal net-like structure.

25. The method according to any of claims 16 to 24, wherein the second expandable member (7) is bonded to the first expandable and contractible member (B) in that
either
the second expandable member (7) is heated to a temperature beyond its melting temperature and after melting of at least a part of the surface of the second expandable member (7) is then pressed onto the inner surface (10) of the first expandable and contractible member (B);
or
the first expandable and contractible member (B) is heated to a temperature above its melting temperature until at least a part of the surface of the first expandable and contractible member (B) is melted before the second expandable member (7) is pressed onto the inner surface (10) of the first expandable and contractible member (B);
or
the first expandable and contractible member (B) and the second expandable member (7) are heated to a temperature beyond both their melting temperature/s and after melting of at least a part of the surface of both, the second expandable member (7) is then pressed onto the inner surface (10) of the first expandable and contractible member (B).

26. The method according to any of claims 16 to 24, wherein the second expandable member (7) is bonded to the first expandable and contractible member (B) in that an adhesive, preferably a flexible adhesive, is applied
either
onto the second expandable member (7) which is then pressed onto the inner surface (10) of the first expandable and contractible member (B);
or
onto the first expandable and contractible member (B) before the second expandable member (7) is pressed onto the inner surface (10) of the first expandable and contractible member (B);
or
onto the first expandable and contractible member (B) and the second expandable member (7) before the second expandable member (7) is pressed onto the inner surface (10) of the first expandable and contractible member (B).

27. The method according to any of claims 16 to 26, wherein an additional third expandable and contractible member (5) is provided disposed inside the second expandable member (7), by expansion of which the second expandable member (7) is pressed onto the inner surface (10) of the first expandable and contractible member (B).

28. The method according to claim 27, wherein the third expandable and contractible member (5) is consisting of a polymer being highly thermal resistant and not-wettable or showing low wettability.

29. A method of producing a medical device according to claim 2, wherein a second expandable member (7) is disposed on the inner surface (10) of a first expandable and contractible member (B) by expanding a third expandable and contractible member (5) disposed inside the second expandable member (7), thus pressing the second expandable member (7) onto the inner surface (10) of the first expandable and contractible member (B).

30. A method of producing according to claim 29, wherein
either
the second expandable member (7) and/or the first expandable and contractible member (B) are heated up to their melting temperature and the third expandable and contractible member (5) disposed inside the second expandable member (7) is expanded after at least part of the surface of one or both of the second expandable member (7) or the first expandable and contractible member (B) is melted, so that the second expandable member (7) is pressed onto the inner surface (10) of the first expandable and contractible member (B);
or
an adhesive, preferably a flexible adhesive is applied onto the second expandable member (7) and/or the first expandable and contractible member (B) and the third expandable and contractible member (5) disposed inside the second expandable member (7) is expanded, so that the second expandable member (7) is pressed onto the inner surface (10) of the first expandable and contractible member (B).

31. A method of producing a medical device according to claim 3, wherein a second expandable member (7) is disposed on the outer surface (11) of a first expandable and contractible member (B) by pulling the second expandable member (7) over at least a partial area of the outer surface (11) of the first expandable and contractible member (B) and optionally bonding the second expandable member (7) to the surface (11) of the first expandable and contractible member (B) by melting or gluing with an adhesive.
